(19) Europäisches Patentamt European Patent Office Office européen des brevets

(11) **EP 3 691 572 B1**

(12) **EUROPEAN PATENT SPECIFICATION**

(45) Date of publication and mention of the grant of the patent:
**21.12.2022 Bulletin 2022/51**

(21) Application number: **17838081.2**

(22) Date of filing: **06.10.2017**

(51) International Patent Classification (IPC):
**A61F 2/30** *(2006.01)*      **A61F 2/34** *(2006.01)*
**A61F 2/40** *(2006.01)*

(52) Cooperative Patent Classification (CPC):
**A61F 2/34; A61F 2/30721; A61F 2/4081;**
A61F 2002/30242; A61F 2002/30245;
A61F 2002/30535; A61F 2002/30574;
A61F 2002/30593; A61F 2002/30649;
A61F 2002/30663; A61F 2002/3208;
A61F 2002/3233; A61F 2002/3443

(86) International application number:
**PCT/IT2017/000217**

(87) International publication number:
**WO 2019/069332 (11.04.2019 Gazette 2019/15)**

(54) **IMPROVED PROSTHESIS COMPONENT, SO-CALLED LINER, FOR AN ACETABULAR OR GLENOID CUP**

VERBESSERTE PROTHESENAUSKLEIDUNG, FÜR EINE HÜFT- ODER GELENK-PFANNEN

COMPOSANT DE PROTHÈSE AMÉLIORÉ POUR UNE BOURRELET COTYLOÏDIEN OU GLÉNOIDE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(43) Date of publication of application:
**12.08.2020 Bulletin 2020/33**

(73) Proprietor: **Limacorporate S.p.A.**
**33038 San Daniele Del Friuli (UD) (IT)**

(72) Inventor: **SBAIZ, Fausto**
**33033 Codroipo (UD) (IT)**

(74) Representative: **Ferreccio, Rinaldo**
**Botti & Ferrari S.p.A.**
**Via Cappellini, 11**
**20124 Milano (IT)**

(56) References cited:
**US-A1- 2007 106 390     US-A1- 2014 039 638
US-A1- 2017 202 671     US-B2- 7 682 398**

**Description**

Technical Field

The present invention concerns an improved prosthesis component of the so-called liner type, constrained inside an acetabular cup or a glenoid cup of an orthopedic prosthesis and intended to receive a spherical joint of a femoral or humeral artificial head, said component being substantially hemispherical-shaped with a base defining the lower edge of the liner.

More particularly, but not exclusively, the prosthesis component of the present invention can be incorporated in an orthopedic prosthesis of the femoral or humeral type without this representing a limit to the Applicant's rights. However, the following description will be made with reference to a femoral prosthesis with the sole aim of simplifying the exposure thereof.

Background Art

As it is well known in this technical field, the femur head replacement is a hip surgical operation in which a femoral joint is replaced by a prosthesis. The prosthesis can be partial, in the sense that just the femur head (endoprosthesis) is replaced, or total, in the sense that also the acetabulum, i.e. the hip cuff in which the femur head rotates, is replaced; both types can be biarticular.

The reasons why this operation is performed are multiple, for example for treating a hip or femur neck traumatic fracture or femur head or acetabulum degenerations or still for treating serious forms of rheumatoid or tumoral arthritis. The hip operation is obviously undergone when other medical or physiotherapy therapies have failed. The complexity and features of the prosthetic component are proportional to the complexity of the anatomical and kinematic defect to be treated.

An orthopedic prosthesis for replacing a complete femoral joint of the hip at least comprises an acetabular cup, which is inserted into the natural cotyle, and an artificial femoral head, substantially spherical-shaped, made of metallic or ceramic material.

The femoral head is anchored to the femur with modalities known in the field, for example through a stem inserted in the medullary canal.

More particularly, the acetabular cup has the shape of a hemispherical bell, usually made of a metal alloy, inside which there is an insert or liner having a conjugated shape and defining a seat for receiving an artificial femoral head.

This insert can be made of a material different with respect to the acetabular cup, for example a synthetic-plastic material like polyethylene or a cobalt-chromium-molybdenum metal alloy different from the metal alloy which the acetabular cup is made of, or still a ceramic material.

The prior art suggests various technical solutions in order to make the insert, which defines the seat for receiving the artificial femoral head, cohesive with the acetabular cup, but these solutions lie outside the present invention.

Instead, the invention concerns an acetabular cup or an insert that could be defined as retentive in the sense that it has structural features such as to counteract the possible dislocation of the artificial head held inside the acetabular cup or the liner.

A first solution proposed by the prior art to achieve this objective is described for instance in US patent n° 7,682,398 B2, wherein an acetabular liner having a circle 36 with a variable edge at the opening 50 of the internal concave surface is described. This edge also has a projection 26 with a chamfered profile connected to said circle.

This circle increases the equatorial edge of the liner by a short cylindrical tract before converging into the projection 26. Though advantageous under several aspects, this solution excessively increases the liner's height and does not allow obtaining great freedom in the step of the artificial head insertion.

Another solution is described in US patent application n° US 2007/106390 to Zimmer, which discloses a liner of the constraining type wherein two opposite projections occupy the missing hemisphere of the hemispherical form of the liner. This solution as well, though advantageous under several aspects, excessively modifies the liner conformation and leaves little freedom to the prosthesis artificial head insertion.

For instance, in US patent n° 7,192,449 B1 to Orthopaedic Reserch Institute Inc., a prosthesis comprising an acetabular cup, an insert or liner and a retainer ring, which is fixed to the circumferential edge of the cup to hold in position the liner, is described.

In US patent application n° 2010/0174380 A1 a liner held inside an acetabular cup through a further intermediate element having a threaded pin that passes through a hole of the acetabular cup is described.

Document US2014039638 (A1) relates to an acetabular prosthesis generaly for implantation in an acetabulum and the surrounding pelvis, which includes an acetabular cup having a substantially concave inner surface and a substantially convex outer surface; one trial shell or a collection of trial shells are provided to trial a range of motion of the hip joint before implanting a shell prosthesis into the acetabular prosthesis.

Document US2017202671 (A1) relates to a system including a constrained acetabular insert, a dual mobility liner, and a femoral head. The constrained acetabular insert has its perimeter extending beyond hemisphere and the dual mobility liner has its perimeter extending beyond hemisphere and configured to tilt and rotate within the constrained acetabular insert.

Document US7682398 (B2) relates to an acetabular shell liner, and particularly to a constrained liner, having a variable rim surface geometry to improve the range of motion of a femoral component within the liner and decrease the incidence of dislocation and subluxation.

Though advantageous under various aspects, all these prior art solutions have a common drawback due to the fact that, in order to properly hold a spherical joint of the prosthesis artificial head, additional components are needed, such as for instance the above retainer rings, or it is necessary to structure the liner with projections that make its use less versatile.

The technical problem underlying the present invention is to conceive a prosthesis component, in particular an acetabular cup or a liner constrained inside an acetabular cup or a glenoidal cup of an orthopedic prosthesis having structural and functional features as to allow containing, effectively and alone, a prosthesis spherical joint, without using accessory or additional components such as, for instance, a retainer ring, features which still limit the prior art solutions.

Another aim of the invention is to allow the articulation of the prosthesis spherical joint and meanwhile constraining the various prosthesis components to each other, preventing a joint decoupling, namely a dislocation.

A further aim of the invention is to conceive a liner constrained inside an acetabular cup or a glenoidal cup of an orthopedic prosthesis having structural and functional features as to allow the coupling of the above liner with a dual-mobility head that is typical of the current offer of orthopedic standards.

Disclosure of the invention

The solution idea underlying the present invention is to provide a lip projecting beyond the equatorial line of the hemispherical form of the liner by at least one tract of the base of the hemispherical form so as to receive and hold a dual-mobility head of an orthopedic prosthesis. According to said solution idea, the technical problem is solved by a prosthesis component, for example an acetabular or glenoidal cup of an orthopedic prosthesis or a liner constrained inside the cup and intended to receive a spherical joint of an artificial femoral or humeral head, said component being substantially hemispherical-shaped with a base defining the lower edge of the liner, characterized by comprising a lip projecting beyond the base and beyond the equatorial line of the hemispherical form, partially occupying the missing hemisphere of the hemispherical form, to define un undercut edge, as well as an extended portion of said lip by at least one tract of said base to receive and hold a dual-mobility head in the spherical segment inside the component.

Advantageously, the artificial head is a dual-mobility head comprising an outermost spheroidal body, internally hollow, which internally receives a spherical joint and in that said lip is extended from a circumferential edge of the base.

In an alternative embodiment, said extended portion of said lip is extended in the circumferential direction by at least one tract which is not greater than half the base.

The lip is preferably continuous.

Alternatively, the lip is crenelated and has tracts spaced by spaces or interruptions that correspond to portions lowered towards the base or coinciding therewith.

In any case, the lip is structurally integral with the liner, being made in one piece with it.

Substantially, the lip is made of the same material as the one the liner is made of.

Advantageously, the internal part of the liner is a spherical segment with a hemisphere completely hollow and intended to receive the dual-mobility head of said prosthesis.

In other words, the dual-mobility head comprises an outermost spheroidal body, internally hollow, which internally receives a spherical joint.

The features and advantages of the prosthesis component according to the invention will result from the following description of an embodiment only given for indicative and non-limiting purposes, with reference to the figures of the appended drawings.

Brief description of drawings

- Figure 1 shows a perspective and schematic view of a complete orthopedic prosthesis incorporating a prosthesis component made according to the present invention to receive a dual-mobility head of an orthopedic prosthesis;

- Figure 2 shows a perspective view of a humeral prosthesis component according to the present invention applied to a shoulder;

- Figures 3 e 4 show respective lateral and section views of a prosthesis component made according to the invention as monobloc cup;

- Figure 5 shows a perspective view of a femoral prosthesis component according to the present invention applied to an acetabulum seat of a basin;

- Figure 6 shows a section view of a prosthesis of figure 1 applied in the acetabular seat of figure 3;

- Figure 7 shows a perspective and schematic view from the bottom of the prosthesis component of figure 1, which receives a dual-mobility head of an orthopedic prosthesis;

- Figure 8 shows a perspective view of the prosthesis component of figure 1 in a first version suitable for being used as femoral prosthesis component of figure 5;

- Figure 9 shows a section view taken along the line A-A of the component of figure 8;

- Figures 10 e 11 show respective lateral and section views of a prosthesis component made according to the invention as liner insert for a modular cup;

- Figure 12 shows a view from the bottom of the insert of figure 10;

- Figures 13, 14 and 15 show respective section views on a vertical plane of further embodiments of the liner insert of figure 10;

- Figures 16 to 19 show respective lateral and section views of the same prosthesis component in the moment when it receives another component of an orthopedic prosthesis, in particular a dual-mobility head.

Detailed description

With reference to such figures, 1 and 1' globally and schematically indicate a prosthesis component improved according to the present invention to receive a spherical joint 5 of an orthopedic prosthesis 12, for example an artificial femoral or humeral head 10. Advantageously, the head 10 is a dual-mobility head that will be described in greater detail in the following.

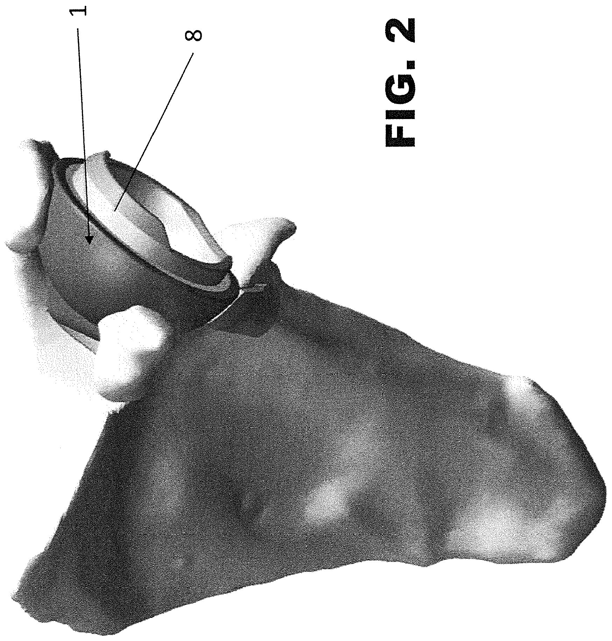

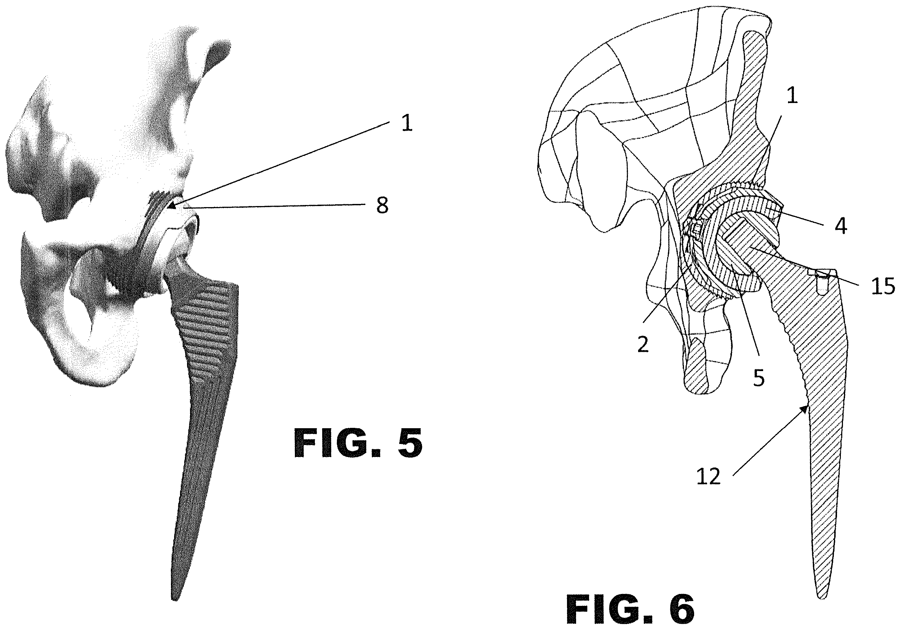

More particularly, as shown in figure 2, the prosthesis component 1 of the present invention can be part of a humeral prosthesis, as shown in figure 2, or part of a femoral prosthesis, as shown in figure 5.

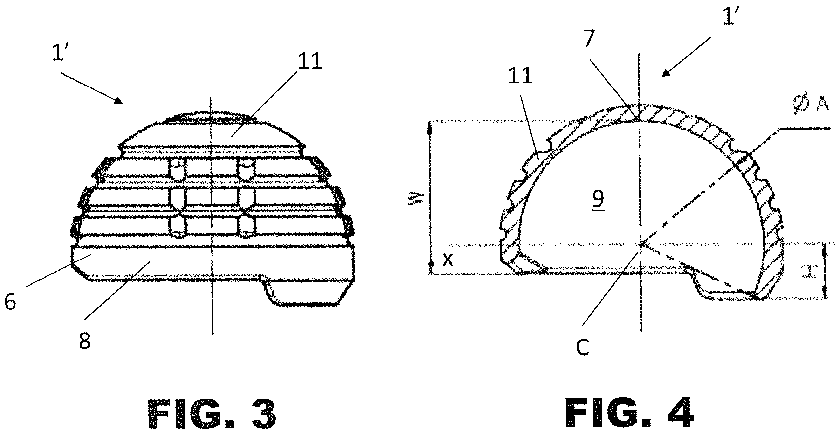

In both cases, the component can be made as a monobloc single piece, indicated in this case with reference number 1'. For example, the component 1' can perform the function of acetabular cup, as shown in figures 3 and 4, in this case we talk about monobloc cup.

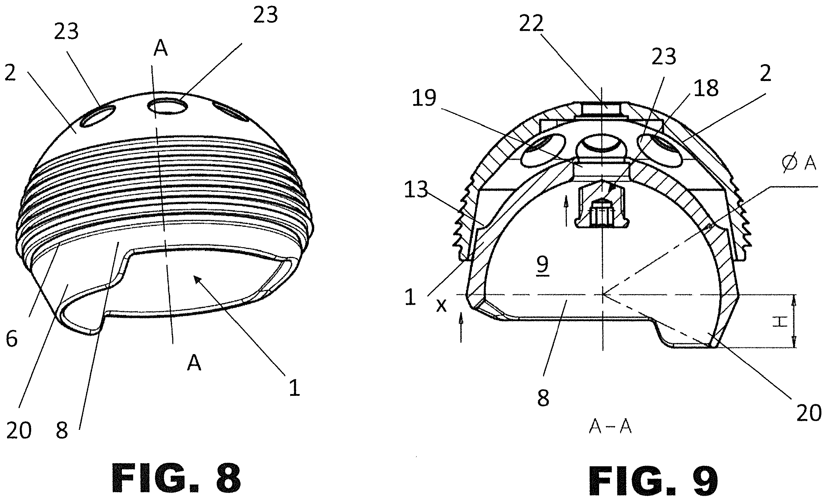

Alternatively, the prosthesis component 1 can be structured as insert or liner to be constrained inside an acetabular cup, as shown in figures 8 and 9, in this case we talk about modular cup.

In the following we will focus on the component 1 used as insert, known in the technical field of the present invention as "liner", and in the rest of the description we will refer to it also with this specific technical term.

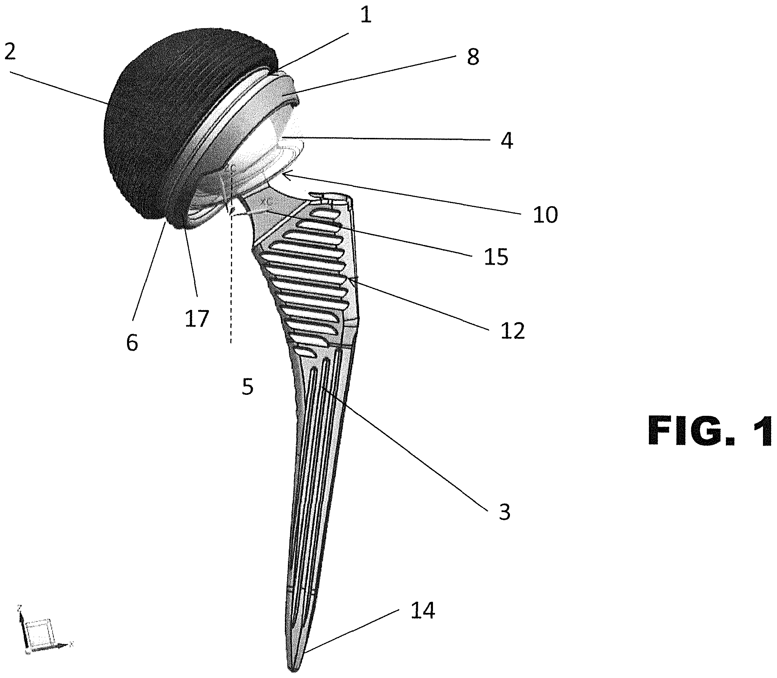

The insert or liner 1 is normally constrained inside an acetabular cup 2 or a glenoidal cup of an orthopedic prosthesis 12, for example a femoral prosthesis shown in figures 1, 5 and 6.

Alternatively, a skilled in the art will well understand how the liner 1 can be applied inside a humeral cup of an analogous orthopedic prosthesis intended to artificially replace a homer head, for example as shown in figure 2.

The following description will be made with reference to the sole femoral prosthesis without this representing a limitation to the Applicant's rights.

The acetabular cup 2 (or glenoidal) is substantially a monobloc bell that is anchored to the natural cotyle of the hip (or, considering the due proportions, in a glenoidal seat of the scapula).

The modalities with which the acetabular cup is constrained or cemented to the cotyle lie outside the present invention and for such modalities one should refer to the prior art of the field.

Therefore, a liner 1 can be constrained to the acetabular cup 2 according to modalities known in the field, which lie outside the structural aspects of the liner of the present invention and which will be described in the following.

For instance, a solution for constraining the liner to the acetabular cup is described in US patent application n° 2010/0234965 A1 to the same Applicant, wherein the liner has an outer surface of prefixed roughness that allows preventing the relative movement between liner and acetabular cup.

For example, as shown in the section of figure 9, the liner 1 is blocked by interference in the cavity of the acetabular cup 2 and has an upper hole 19 through which a fastening screw 18 in inserted, engaging in an upper threaded hole 22 obtained in the cup 2.

However, precisely the modalities with which the liner 1 is constrained or constrainable to the acetabular cup 2 must all be considered compatible and combinable with the structural features of the liner 1, which will be described in the following. Differently from the prior art solutions, the invention does not require the use of fixing rings.

The liner 1 has an outer surface 11 that can be knurled, irregular or again it can be an outer surface of prefixed roughness that allows preventing the relative movement between liner and acetabular cup 2, for example as described in Italian patent n° IT 1 288 859 B1 to the same Applicant.

Alternatively, at least part of the outer surface 11 of the liner 1' can be affected by streaks or stripes extended according to parallel lines, for example as shown in figure 3, when the liner 1' is structured as monobloc cup.

Still alternatively, the liner 1 can be associated with an acetabular cup 2 equipped with holes 23 that allow the passage of bone screws or stabilization screws, as shown in figure 9, or again it can be equipped with pins intended to engage corresponding seats inside the acetabular cup, in order to constrain it with the acetabular cup 2 for example as described in European patent n° 1 336 394 B1 to the same Applicant.

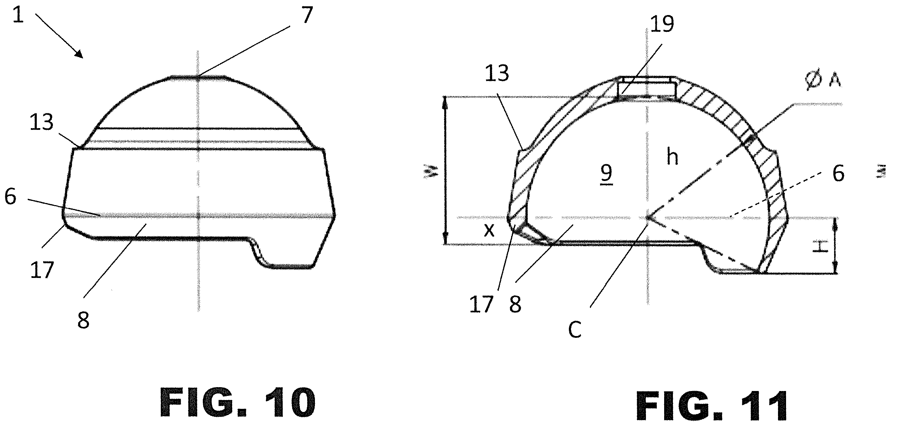

Finally, the liner 1 can have a substantially smooth outer surface, as shown in figure 10, but interested by at least one annular step or relief 13.

Advantageously, regardless the conformation of the outer surface 11, the liner 1 of the present invention essentially has the shape of a spherical shell or ball portion defined by a spherical form delimited by a secant plane X.

In a preferred embodiment, the secant plane X passes through a diameter of the spherical form, namely through an equatorial plane of the spherical form, so that the liner 1 can be defined a hemisphere. Therefore, we can say that the liner 1 has an essentially hemispherical shape.

As defined by the geometry, the circle delimited by the spherical form and by the secant plane X is called "base" of the spherical shell. Therefore, the liner 1 also has a base 6 that can also be considered as lower edge of the liner.

We will see in the following that this reference plane x can also be inclined by a beta angle and distinguish itself from the previously defined equatorial plane.

The radius passing through the center C of the base 6 of the liner 1 is a symmetry axis for the liner 1 and meets the shell at a point that will be called vertex 7. The part of radius comprised between the base 6 and the vertex 7 is the height h of the liner 1.

In this sense the base 6 can be defined as lower edge of the liner 1 if one considers the vertex 7 as upper end. The base 6 belongs to the secant plane X.

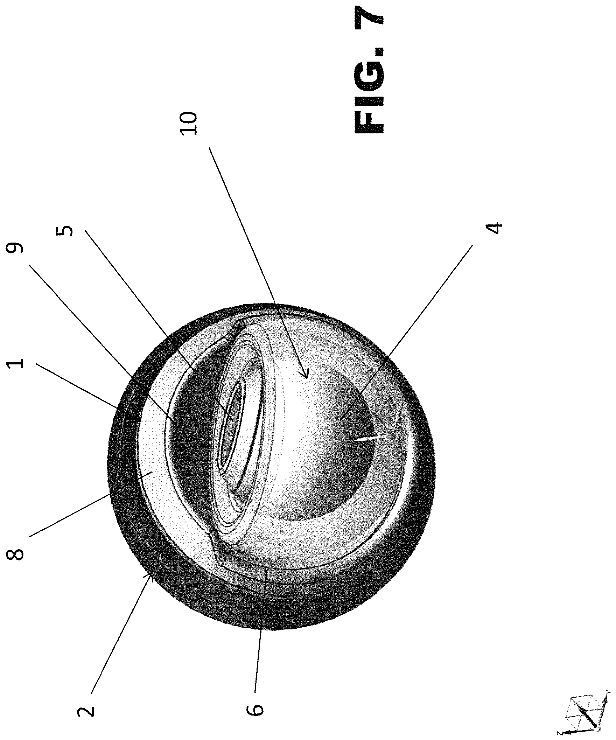

The internal spherical segment 9 of the liner 1, namely the volume comprised between the spherical shell and the secant plane X, is occupied by a prosthesis artificial femoral or glenoidal head 10, as it will be clear form the rest of the description and as well shown in figures 6 and 7.

The prosthesis head 10 is a dual-mobility head and comprises two components one inside the other, and namely a spheroidal body 4 internally receiving and covering a spherical joint 5.

The dual mobility essentially comprises a dual joint.

A first joint, also called small joint, is between a spherical head 5, so-called small head, and a covering insert 4 made of yielding material. The materials used for the small joint can be polyethylene, PEEK or ceramic, wherein the head 5 is inserted inside the covering insert 4 and completely mobile therein.

The second joint, called big joint, can be identified between the insert 4 of polyethylene and the liner 1, or the acetabular cup 1' in case of monobloc cup.

With respect to the standard prostheses, what varies is the interface between the cup 2 and the insert 4 made of polyethylene, since, instead of being fixed, is mobile, precisely a second joint.

Advantageously, according to the present invention, the liner 1 has a lip 8 projecting beyond the base 6.

In other words, the lip 8 slightly projects beyond the equatorial line represented by the plane x of the hemispherical shape of the liner 1 substantially as if it partially occupied the other missing hemisphere of the hemispherical form.

In other words, the lip 8 represents a substantially undercut containment edge, in the sense that its profile substantially follows the spherical movement of the internal cavity of the liner 1.

The lip 8 is preferably continuous.

Advantageously, still according to the invention, the lip 8 has an extended portion 20 even more projecting with respect to the equatorial line represented by the plane x of the hemispherical form. This extended portion 20 can reach a size or extension H, with respect to the reference plane x.

This extended portion 20 of the lip 8 is circumferentially extended only by at least one tract of the base 6.

More particularly, said extended portion 20 of the lip 8 is extended by at least one tract that is not greater than half the base 6. Preferably, the extended portion 20 is extended by at least one third of the base 6.

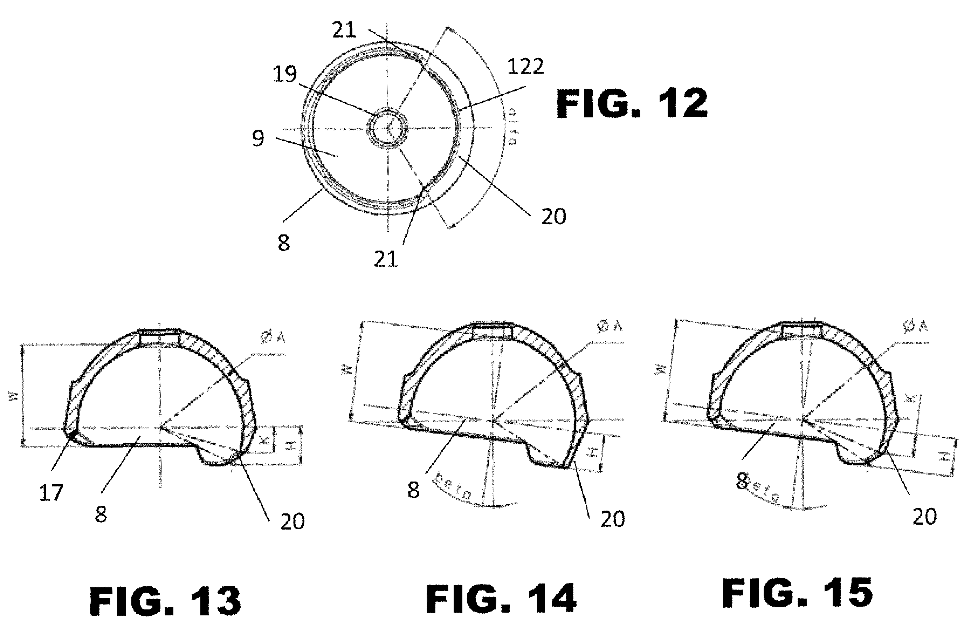

More particularly, as well shown in figure 12, the circumferential extension of the extended portion 20 is essentially defined by an "alfa" angle that is variable between a minimum of 0° and a maximum of 180°.

Obviously, in case of anchoring equal to 0°, it is as if the extended portion 20 did not exist.

In figures 13, 14 and 15 respective sections on vertical plane of three different embodiments of the lip 8 and of the extended portion 20 are schematically shown.

The figures concern three different variants of embodiment of the liner of the present invention that will be respectively defined: open-lip liner; closedlip angled liner and open-lip angled liner.

In these variants of embodiments, the extended portion 20 is connected to the lip 8 through respective arched tracts 21.

Furthermore, the central part 122 of the extended portion 20 can be less extended than the initial part so as to centrally have an arched profile, as shown in figure 12.

In this case a dimensional parameter K can be defined, which represents the maximum extension of the central part 122 of the extended portion 20 with respect to the reference plane x; whereas the dimensional parameter K remains the extension or the maximum projection of the extended portion 20 still with respect to the reference plane x.

In such figures 13, 14, and 15 the inclination angles with respect to the equatorial plane x of the liner 1 and the initials of the dimensional parameters that can be suitably adopted to produce liners perfectly compatible with the principles of the present invention are also represented. In particular, a beta ($\beta$) angle of possible inclination of the reference plane x with respect to the equatorial plane is also indicated.

The dimensional parameters of these embodiments can be summed up in a table in which the relationships between the dimensional parameters are reported in relative terms. Furthermore, we could define these parameters as generic parameters in the left column, more restrictive parameters in the central column and restrictive parameters in the right column.

$$K \leq H$$

$$\text{beta} \leq 20° \qquad \rightarrow \qquad \text{beta} \leq 10° \qquad \rightarrow \qquad \text{beta} \leq 7.5°$$

$$\frac{\text{ØA}}{2} - H \leq W \leq \frac{\text{ØA}}{2} + H \qquad \rightarrow \qquad \frac{\text{ØA}}{2} - \frac{3}{4}H \leq W \leq \frac{\text{ØA}}{2} + \frac{3}{4}H \qquad \rightarrow \qquad \frac{\text{ØA}}{2} - \frac{H}{2} \leq W \leq \frac{\text{ØA}}{2} + \frac{H}{2}$$

$$0° \leq \text{alfa} \leq 180° \qquad \rightarrow \qquad 45° \leq \text{alfa} \leq 160° \qquad \rightarrow \qquad 90° \leq \text{alfa} \leq 140°$$

Alternatively, a skilled in the art can appreciate how the lip 8 can be crenelated, namely produced with tracts spaced by spaces or interruptions that correspond to tracts lowered towards the base 6 or possibly coinciding therewith.

The lip 8 is structurally integral with the liner 1 being made in one piece with it and of the same material as the one the liner is made of, for example a titanium alloy.

Advantageously, the internal part 9 of the liner 1 is a spherical segment with a hemisphere that is completely hollow and intended to receive the dual-mobility head 10 of the prosthesis 12, for example a femoral prosthesis. The prosthesis 12 is conventionally structured with a stem 3 having a sharped distal end 14 and a proximal end 15 equipped with a spherical head 5.

As already described, the invention requires a connection with a prosthetic or humeral femoral head and by congruence with the bone component of the homer or femur; said element, commonly known with the term of humeral or femoral stem and known in orthopedic surgical technique is represented in the figure with reference number 12.

Purely by way of completeness of description, it is noted that such a prosthesis 12 can be conformed with a pointed distal end intended to be inserted into the medullary canal of a long bone, for example a femur or a homer, whereas the proximal end bears a spherical head 5 of an artificial articulation joint.

The prosthesis 12 can be metallic made of an alloy compatible with an implant in the human body, for example made of titanium or titanium alloy or cobalt-chromium. The overall structure of the prosthesis 12 can anyway be different according to the application needs.

Advantageously, according to the present invention, the head 10 is of the dual-mobility type and comprises an outermost spheroidal body 4, which is internally hollow, and an innermost spherical joint 5, as shown in figures 1 and 2.

The spheroidal body 4 is fitted on the spherical joint 5 so as to be able to freely rotate on it with very little clearance.

The spherical joint 5 has a frustoconical cavity 16 that allows constraining it to the proximal end of the stem 3 of the prosthesis 12. Such a joint 5 is a component of the dual-mobility spherical head 10 of the prosthesis 12.

The overall structure of the liner 1 inserted inside the acetabular cup 2 and receiving in turn the spherical joint 5 covered by the spheroidal body 4 of the head 10 of the prosthesis 12 configures a prosthetic joint component of the ball-on-socket type suitable for hip joints or for shoulder joint as well through simple proportional adjustments.

**EP 3 691 572 B1**

This configuration gives the liner of the present invention a definition of constrained liner or captured liner.

The peculiarity of the liner 1 according to the invention is thus that of allowing a substantially fast-snap coupling with a dual-mobility head, or with an analogous product of the orthopedic standard.

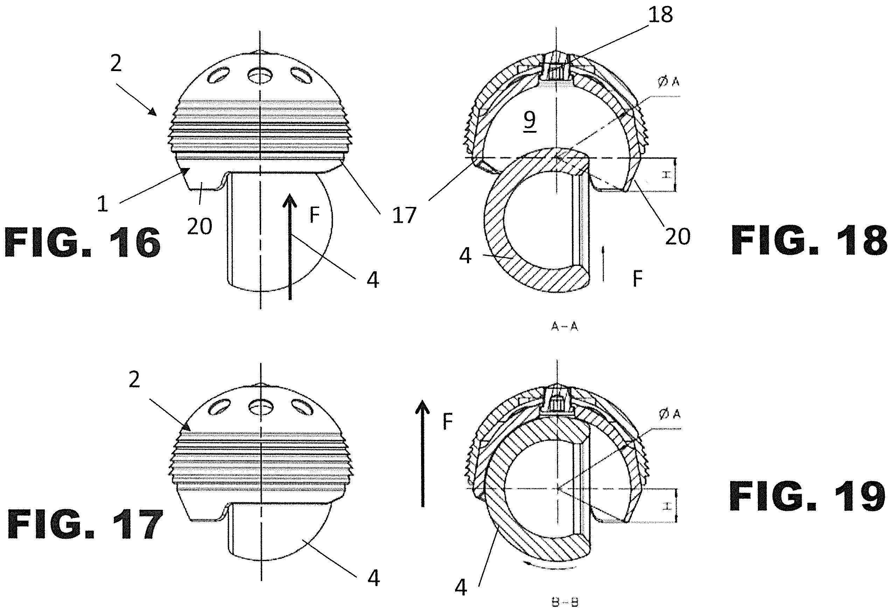

The peculiar shape of the liner 1 with the lip 8 and of its extended portion 20, both projecting beyond the base 6 allows inserting the head 4 in a unique position according to the direction of the arrow F, as shown in figures 16 to 19, which substantially corresponds to the height h of the internal spherical segment or hemispherical cavity 9. Furthermore, the shape of the liner 1 assures the containment of the same head 4 in any other possible angular position thereof without using additional components since the lip 8 represents an undercut edge that in fact prevents the head 10 from exiting from the liner.

The containment of the spheroidal body 4 of the head 10 is mainly made possible precisely by the lower lip 8, as well as by the minimal interference obtained with the extended portion 20. A possible extension of the base 6 by a tract 17 less than one millimeter provides an over-covering such as to ensure a further stability to the head 10.

This configuration can also be alternative and optional, even if shown in figures 10 to 19, wherein the over-equatorial edge or tract 17 does not have a solution of continuity with the base 6, whereas the lip 8 extends therefrom in the direction of the missing hemisphere instead of extending directly from the base 6.

The edge 17 is similar to an equatorial cylindrical extension of less than one millimeter of the base 6 and is present on the whole circumference of the base.

The peculiar shape of the liner 1 of the present invention allows minimizing the so-called joint Range of Motion (ROM), as well as properly positioning the lip 8 and its extension 20 in the most suitable position to the anatomy of the patient.

Though respecting the values of stability to the pull-out of the head, the ROM is maximized for the main joint movements by maintaining an almost hemispherical shape.

The component can be provided for a conical coupling with Metal back, as shown in figure 5, or for cemented implantation in conjunction with Metal back or directly with the anatomic surface.

The prosthesis component according to the invention solves the technical problem and provides several advantages, the first of which is due to the fact that the structure of the liner undergoes a modest structural modification if compared with the alternative prior art solutions, though ensuring an easy insertion of the dual-mobility head, which this kind of liner is specifically intended to and an almost impossible luxation of the head once inserted.

**Claims**

1. Orthopedic prosthesis component (1, 1'), for example an acetabular or glenoidal cup (2) of an orthopedic prosthesis (12), or a liner constrained inside the cup (2), and intended to receive a spherical joint (4, 5) of an artificial femoral or humeral head (10), said component being substantially hemispherical-shaped with a base (6) defining the lower edge of the component (1), **characterized in that** it comprises a lip (8) projecting beyond the base (6) and beyond the equatorial line of the hemispherical form, partially occupying the missing hemisphere of the hemispherical form so as to define an undercut edge, as well as an extended portion (20) of said lip (8) by at least one tract of said base (6) in order to receive and hold a dual-mobility head in the spherical segment (9) inside the component (1, 1').

2. Prosthesis component (1) according to claim 1, **characterized in that** said extended portion (20) of said lip (8) is circumferentially extended by an angle alfa comprised between 0° and 180°.

3. Prosthesis component (1) according to claim 1, **characterized in that** said lip (8) is preferably continuous.

4. Prosthesis component (1) according to claim 1, **characterized in that** said lip (8) is structurally integral with the component (1) since it is made in one piece with it.

5. Prosthesis component (1) according to claim 1, **characterized in that** said lip (8) is made of the same material as of the component (1).

6. Prosthesis component (1) according to claim 1, **characterized in that** the internal part (9) of the component (1) is a spherical segment with a hemisphere that is completely hollow and intended to receive said dual-mobility head (10) of said prosthesis (12).

7. Prosthesis component (1) according to claim 1, **characterized in that** the dual-mobility head (10) can be inserted into the cavity (9) of the component (1) according to a direction (F) corresponding to the height (h) of the hemispherical form of said cavity (9).

8. Prosthesis component (1) according to claim 1, **characterized in that** some dimensional parameters of the component are selected according to the ranges of values reported in the following table wherein:

W is the overall height of the hemispherical portion of the component including the (8);
øA is the diameter of the hemispherical portion;
H is the maximum extension of the extended portion (20);
K is the extension of a central part (122) of the extended portion (20);
alfa is a circumferential extension of the extended portion (20);
beta is an inclination angle of a reference plane (x) with respect to an equatorial plane;
with $K \leq H$ and $W \leq (\emptyset A/2) + H$.

**Patentansprüche**

1. Orthopädische Prothesenkomponente (1, 1'), beispielsweise eine Hüftgelenkpfanne oder Schulterpfanne (2) einer orthopädischen Prothese (12), oder eine Auskleidung, die in der Pfanne (2) festgelegt ist, die dafür vorgesehen ist, ein Kugelgelenk (4, 5) eines künstlichen Hüftkopfes oder Oberarmkopfes (10) aufzunehmen, wobei die Komponente im Wesentlichen halbkugelförmig ausgebildet ist und eine Basis (6) aufweist, die den unteren Rand der Komponente (1) bildet,
**dadurch gekennzeichnet ist, dass** sie eine Lippe (8) aufweist, die über die Basis (6) und über die Äquatoriallinie der halbkugelförmigen Form hinaus vorsteht und die teilweise die fehlende Halbkugel der halbkugelförmigen Form einnimmt, um eine hinterschnittene Kante zu bilden, sowie einen verlängerten Bereich (20) der Lippe (8) um mindestens ein Stück der Basis (6) aufweist, um einen Kopf mit dualer Mobilität in dem kugelförmigen Segment (9) im Inneren der Komponente (1, 1') aufzunehmen und zu halten.

2. Prothesenkomponente (1) nach Anspruch 1,
**dadurch gekennzeichnet, dass** der verlängerte Bereich (20) der Lippe (8) in Umfangsrichtung um einen Winkel alfa verlängert ist, der zwischen 0° und 180° liegt.

3. Prothesenkomponente (1) nach Anspruch 1,
**dadurch gekennzeichnet, dass** die Lippe (8) vorzugsweise kontinuierlich ist.

4. Prothesenkomponente (1) nach Anspruch 1,
**dadurch gekennzeichnet, dass** die Lippe (8) strukturell in die Komponente (1) integriert ist, indem sie einstückig mit dieser hergestellt ist.

5. Prothesenkomponente (1) nach Anspruch 1,
**dadurch gekennzeichnet, dass** die Lippe (8) aus dem gleichen Material wie die Komponente (1) hergestellt ist.

6. Prothesenkomponente (1) nach Anspruch 1,
**dadurch gekennzeichnet, dass** der innere Teil (9) der Komponente (1) ein kugelförmiges Segment mit einer Halbkugel ist, die vollständig hohl ist und dafür vorgesehen ist, den duale Mobilität aufweisenden Kopf (10) der Prothese (12) aufzunehmen.

7. Prothesenkomponente (1) nach Anspruch 1,
**dadurch gekennzeichnet, dass** der Kopf (10) mit dualer Mobilität in den Hohlraum (9) der Komponente (1) in Übereinstimmung mit einer Richtung (F) eingesetzt werden kann, die der Höhe (h) der halbkugelförmigen Form des Hohlraums (9) entspricht.

8. Prothesenkomponente (1) nach Anspruch 1,
**dadurch gekennzeichnet, dass** einige Abmessungsparameter der Komponente gemäß den in der folgenden Tabelle angegebenen Wertebereichen ausgewählt sind, wobei:

W die Gesamthöhe des halbkugelförmigen Bereichs der Komponente einschließlich der Lippe (8) ist;
øA der Durchmesser des halbkugelförmigen Bereichs ist;
H die maximale Ausdehnung des verlängerten Bereichs (20) ist;
K die Ausdehnung eines zentralen Teils (122) des verlängerten Bereichs (20) ist;
alfa eine umfangsmäßige Ausdehnung des verlängerten Bereichs (20) ist;

beta ein Neigungswinkel einer Referenzebene (x) in Bezug auf eine Äquatorialebene ist;
mit K ≤ H und W ≤ (ØA / 2) + H.

## Revendications

1. Composant de prothèse orthopédique (1, 1'), par exemple une cupule cotyloïdienne ou glénoïde (2) d'une prothèse orthopédique (12), ou une doublure contrainte à l'intérieur de la cupule (2), et destinée à recevoir un joint sphérique (4, 5) d'une tête fémorale ou humérale artificielle (10), ledit composant étant sensiblement de forme hémisphérique avec une base (6) définissant le bord inférieur du composant (1), **caractérisé en ce qu'**il comprend une lèvre (8) faisant saillie au-delà de la base (6) et au-delà de la ligne équatoriale de la forme hémisphérique, occupant partiellement l'hémisphère manquant de la forme hémisphérique de façon à définir un bord de dégagement, ainsi qu'une portion étendue (20) de ladite lèvre (8) par au moins une voie de ladite base (6) afin de recevoir et maintenir une tête à double mobilité dans le segment sphérique (9) à l'intérieur du composant (1, 1').

2. Composant de prothèse (1) selon la revendication 1, **caractérisé en ce que** ladite portion étendue (20) de ladite lèvre (8) est étendue de manière circonférentielle d'un angle alpha compris entre 0° et 180°.

3. Composant de prothèse (1) selon la revendication 1, **caractérisé en ce que** ladite lèvre (8) est de préférence continue.

4. Composant de prothèse (1) selon la revendication 1, **caractérisé en ce que** ladite lèvre (8) est structurellement d'un seul tenant avec le composant (1) puisqu'elle est faite d'une seule pièce avec celuici.

5. Composant de prothèse (1) selon la revendication 1, **caractérisé en ce que** ladite lèvre (8) est faite du même matériau que le composant (1).

6. Composant de prothèse (1) selon la revendication 1, **caractérisé en ce que** la partie interne (9) du composant (1) est un segment sphérique avec un hémisphère qui est complètement creux et destiné à recevoir ladite tête à double mobilité (10) de ladite prothèse (12).

7. Composant de prothèse (1) selon la revendication 1, **caractérisé en ce que** la tête à double mobilité (10) peut être insérée dans la cavité (9) du composant (1) selon une direction (F) correspondant à la hauteur (h) de la forme hémisphérique de ladite cavité (9).

8. Composant de prothèse (1) selon la revendication 1, **caractérisé en ce que** certains paramètres de dimension du composant sont choisis selon les plages de valeurs indiquées dans le tableau suivant dans lequel :
W est la hauteur globale de la portion hémisphérique du composant incluant la (8):

   ØA est le diamètre de la portion hémisphérique ;
   H est l'étendue maximale de la portion étendue (20) ;
   K est l'étendue d'une partie centrale (122) de la portion étendue (20) ;
   alpha est une étendue circonférentielle de la portion étendue (20) ;
   bêta est un angle d'inclinaison d'un plan de référence (x) par rapport à un plan équatorial ;
   avec K ≤ H et W ≤ (ØA /2 ) + H.

**FIG. 1**

EP 3 691 572 B1

FIG. 2

FIG. 4

FIG. 3

FIG. 6

FIG. 5

FIG. 7

FIG. 9

FIG. 8

FIG. 11

FIG. 10

**FIG. 12**

**FIG. 13**

**FIG. 14**

**FIG. 15**

**FIG. 16**

**FIG. 17**

**FIG. 18**

A—A

**FIG. 19**

B—B

EP 3 691 572 B1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 7682398 B2
- US 2007106390 A, Zimmer
- US 7192449 B1
- US 2014039638 A1
- US 2017202671 A1
- US 20100234965 A1
- IT 1288859 B1
- EP 1336394 B1